Europäisches Patentamt

European Patent Office

Office européen des brevets

(11) Publication number: **0 421 109 A2**

# EUROPEAN PATENT APPLICATION

(21) Application number: 90116150.5

(22) Date of filing: 23.08.90

(51) Int. Cl.5: **C12Q 1/37**, C12N 15/57, C12N 15/00, C12N 1/21, //C12N15/62,(C12N1/21, C12R1:19)

The applicant has subsequently filed a sequence listing and declared, that it includes no new matter.

(30) Priority: **11.09.89 US 405609**

(43) Date of publication of application:
**10.04.91 Bulletin 91/15**

(84) Designated Contracting States:
**AT BE CH DE DK ES FR GB GR IT LI LU NL SE**

(71) Applicant: **AMERICAN CYANAMID COMPANY**
**1937 West Main Street P.O. Box 60**

**Stamford Connecticut 06904-0060(US)**

(72) Inventor: **Baum, Ellen Z.**
**18 Stubbe Drive**
**Stony Point, New York(US)**
Inventor: **Gluzman, Yakov**
**24 Peach Tree Place**
**Upper Saddle River, New Jersey(US)**

(74) Representative: **Wächtershäuser, Günter, Dr.**
**Tal 29**
**W-8000 München 2(DE)**

(54) Screening methods for protease inhibitors.

(57) The invention provides methods to screen for inhibitors of proteases. The first method involves the construction of a plasmid system such that expression of a protease is toxic to a bacterial strain. Compounds of interest may then be screened for those which inhibit the toxic protease and thereby prevent killing of the bacterial strain. Inhibition is detected by successful transformations of the bacterial strain by the plasmid coding for the protease. The second method involves the construction of a fusion protein which contains a protease cleavage site inserted into a protein whose activity may be assayed. Compounds are screened for protease inhibition, which is detected by assaying for a change in color, light or activity of the protein. HIV protease and polio 3C protease are examples of proteases that may be used in these methods.

FIG. 1

## SCREENING METHODS FOR PROTEASE INHIBITORS

### BACKGROUND OF THE INVENTION

Proteases are a class of proteins some of whose members have been implicated in human diseases. For example, human renin is an aspartyl protease which has been implicated in hypertension through its role in the formation in plasma of the vasoactive peptide angiotensin (Bibliography entry 1). The proteases trypsin and elastase are involved in the onset of emphysema (2,3). Of particular interest is the protease associated with the human immunodeficiency virus ("HIV"). Polio protease is also of interest.

The primary translation products of HIV include polyprotein precursors encoded by the gag and pol genes (4). The gag gene encodes the capsid proteins; the pol gene encodes the viral enzymes, comprising a 10 kd viral protease, a 66 kd reverse transcriptase and a 34 kd endonuclease (5). The production of these proteins is dependent upon the cleavage of these polyproteins by the viral protease, which is generated by autodigestion of the pol polyprotein.

As a viral-specific enzyme, the protease is an attractive target for drugs for the inhibition of viral replication, and has become the focus of intensive investigation. The pol sequence (nucleotides 1833-2129) produces a mature 99 amino acid protease (6), with proline at the amino terminus and phenylalanine at the carboxy-terminus (7; Graves). The 99 amino acid protease is homologous to the aspartic protease family (8), and is inhibited by pepstatin A (9). Recombinant protease mutated at the catalytic Asp 25 residue is enzymatically inactive in bacterial expression systems and in vitro (5,6), and virus mutated at this site is non-infectious (6). The protease has recently been purified from E. coli and crystallized (10).

At present, there is an urgent need to develop therapeutic agents against acquired immune deficiency syndrome ("AIDS"), which is believed to be caused by HIV. An agent which is selective for HIV protease might serve to inhibit HIV replication and thus be efficacious in the treatment of AIDS. There is thus a need for methods to screen for such inhibitors of HIV protease.

Existing methods for assaying HIV protease utilize polyprotein or oligopeptide substrates, and they require gel electrophoresis or high performance liquid chromatography to ascertain the presence of cleavage products (11-16). Other methods recently developed for multiple samples include resonance energy transfer (17) and release of radioactive products (18). These methods are not easily adapted for assaying protease activity in large numbers of samples.

Accordingly, it is an object of this invention to develop screening methods for identifying inhibitors of proteases.

It is a particular object of this invention to develop screening methods for identifying inhibitors of HIV protease.

It is a further object of this invention to develop screening methods for identifying inhibitors of polio protease.

### SUMMARY OF THE INVENTION

The present invention provides methods for screening for compounds which inhibit protease enzymes. In one embodiment of the invention, the method is based first upon the construction of a plasmid having a DNA sequence which encodes a protease under the control of a T7 promoter. Next, a bacterial expression strain is selected which is not transformed by the plasmid because of the toxicity of the protease to the bacterial strain. A compound to be screened for inhibitory activity is then added together with the plasmid to the bacterial strain. If the bacterial strain is not killed and produces transformants, the compound is identified as an inhibitor of the protease.

In particular, the first embodiment of the present invention provides plasmid constructs designated pPDB16 and pMae2 which contain a DNA sequence coding for HIV protease. These plasmids do not yield transformants in an E. coli strain designated BL21(DE3). Thus, expression of HIV protease from the plasmid appears to be toxic to this E. coli strain. This discovery suggests a method for identifying agents which inhibit HIV protease. A similar plasmid construct, designated pPolio protease 3C, is described for polio protease, which may be used to screen for inhibitors of polio protease.

In another aspect of the first embodiment of the invention, a method for screening for compounds which inhibit HIV protease is described. A plasmid is constructed having a DNA sequence which encodes HIV protease under the control of a T7 promoter. Next, a bacterial strain is selected which is transformed by the plasmid and is then killed by the induction of protease synthesis by an inducing agent. A compound to be screened for inhibitory activity is then added together with the inducing agent to the transformed bacterial strain. If the induced cells are not killed, the compound is identified as an inhibitor of HIV protease.

In the second embodiment of the invention, the method involves the selection of a protein which is capable of being assayed for a change in color, light or activity when combined with a protease enzyme in the presence or absence of a potential inhibitor compound.

Next, a heterologous DNA sequence coding for a peptide which includes a protease enzyme cleavage site is inserted into the gene coding for the selected protein. The fused gene is inserted into a suitable expression system and is combined with the protease enzyme which recognizes and cleaves the peptide coded for by the heterologous DNA sequence embedded within the fusion protein, such that the protease enzyme cleaves at the protease enzyme cleavage site, which in turn inactivates the expressed fusion protein.

This loss of activity is monitored by means of a color change, light change or by directly monitoring the loss of activity. A compound to be screened is included in the assay system and is identified as an inhibitor of the protease enzyme if the protein is not cleaved and remains active when monitored by any of the methods described previously.

In particular, the second embodiment of this invention provides a plasmid construct designated p1 + IQSau, which includes the gene for $\beta$-galactosidase, into which is inserted a DNA sequence coding for a decapeptide HIV protease cleavage site. When expressed, this fusion protein retains $\beta$-galactosidase activity. HIV protease cleaves and inactivates the fusion protein.

Potentially inhibitory compounds of HIV protease are screened by monitoring $\beta$-galactosidase activity, such that maintenance of $\beta$-galactosidase activity indicates that the compound prevents cleavage and inhibits HIV protease.

A similar construct, designated plasmid pZM3CSau, includes the gene coding for $\beta$-galactosidase into which is inserted a DNA sequence coding for a polio 3C protease cleavage site. This construct is used to screen for compounds which inhibit polio 3C protease using procedures similar to those described previously for HIV protease.

## BRIEF DESCRIPTION OF THE DRAWINGS

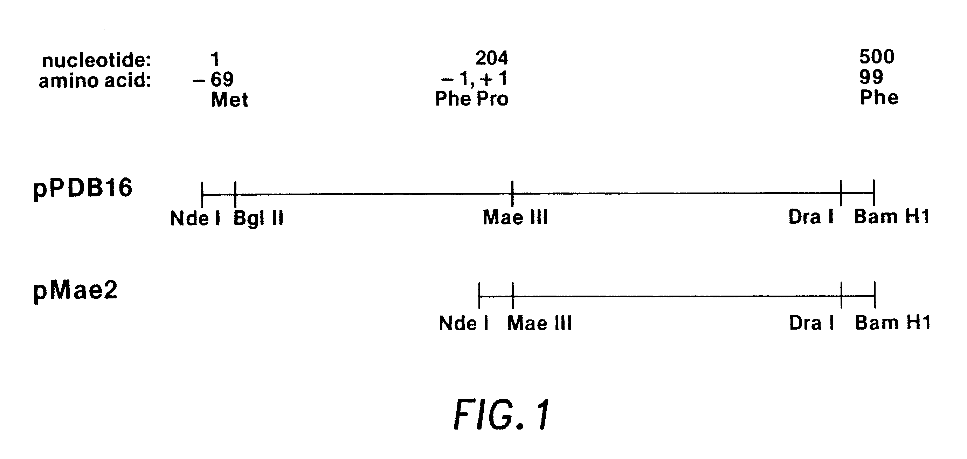

Figure 1 depicts the HIV protease constructs pPDB16 and pMae2. Construct pPDB16 encodes the 18 kd protease precursor under the control of the T7 promoter. The BglII-DraI fragment from λBH5 (4), corresponding to nucleotides 1418-1899 of the HIV pol gene, is subcloned into the NdeI-BamHI sites of the previously described expression vector (19), using synthetic oligonucleotides. The upstream oligonucleotides begin with an NdeI restriction site, providing an in-frame translational start codon, and regenerate nucleotides 1406-1417. The downstream oligonucleotides regenerate nucleotides 1900-1906, followed by two in-frame stop codons and a BamHI restriction site. For clarity, base A of the initiation codon is defined as nucleotide (nt) 1. Amino acids +1 to +99 represent the mature 10 kd protease liberated by autodigestion at the indicated Phe-Pro bond (7). pMae2 encodes the mature 10 kd protease. Its construction is similar to PPDB16, except that the upstream oligonucleotides regenerate from nucleotide 1610 to the MaeIII restriction site.

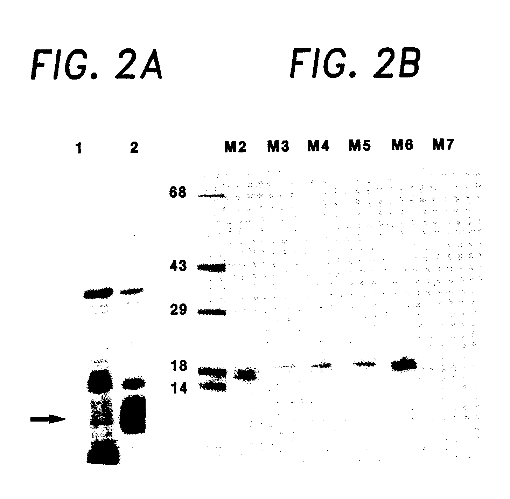

Figure 2 depicts the expression of HIV protease in E. coli . Panel A: Protease synthesis in BL21(DE3)-pLysS cells harboring pPDB16. Proteins are labelled with $^{35}$S methionine as described (19), followed by electrophoresis and autoradiography. Lane 1, no isopropyl-$\beta$-D-thiogalactopyranoside ("IPTG"). Lane 2, 1mM IPTG. The arrow denotes the mature 10 kd protease. Panel B: Protease synthesis in BL21(DE3) survivors harboring mutagenized pPDB16. The numbers above each lane correspond to the clone designations in Tables II and III. Molecular weight markers are shown on the left (68 kd - bovine serum albumin; 43 kd -ovalbumin; 29 kd - carbonic anhydrase; 18 kd -beta-lactoglobulin; 14 kd - lysozyme).

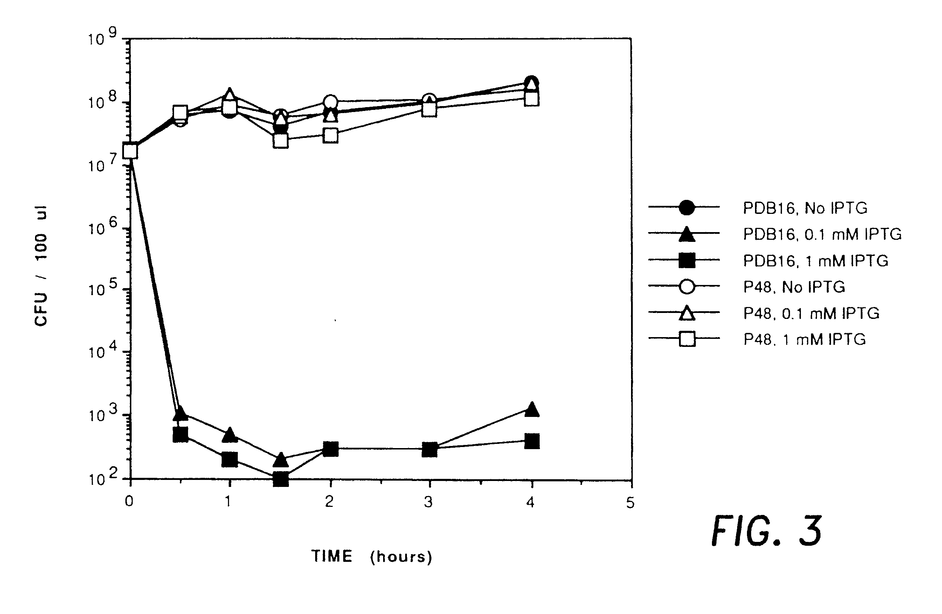

Figure 3 depicts the killing of BL21(DE3)pLysS upon induction of HIV protease synthesis with IPTG. Cells harboring plasmid encoding wild type (pPDB16) or mutant (pP48) protease precursor are grown to 0.5 absorbance units (600 nm), and IPTG is added to the indicated concentrations. At the indicated times, an aliquot of each culture is removed, and dilutions are plated on LB plates containing kanamycin and chloramphenicol. After overnight incubation, colony forming units ("CFU") per 100 $\mu$l of culture are determined.

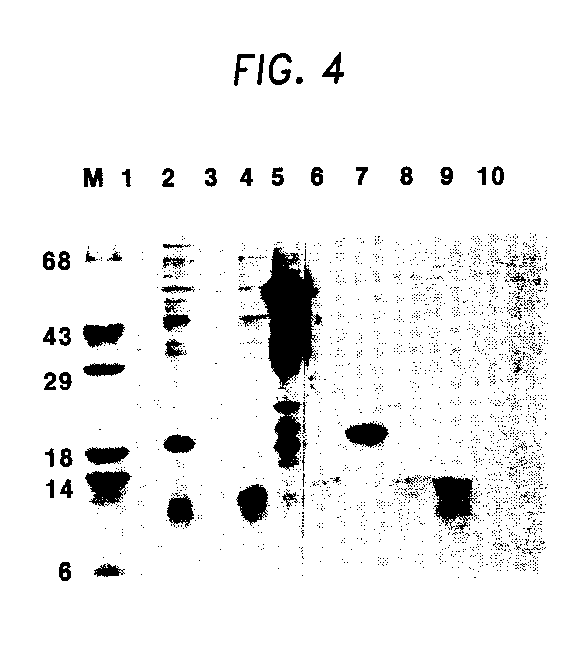

Figure 4 depicts the comparison of amounts of wild-type and mutant protease production. BL21(DE3)-

pLysS cells harboring the indicated plasmids are induced with 1mM IPTG, and proteins are labelled with $^{35}$S methionine as described (19). Whole cell extracts (lanes 1-5), and immunoprecipitates (lanes 6-10) from antiserum raised to amino acids 34-46 of the mature protease, are subjected to electrophoresis and autoradiography. Lanes 1 and 6, pPDB16. Lanes 2 and 7, pP48. Lanes 3 and 8, pMae2. Lanes 4 and 9, pP48Mae6. Lanes 5 and 10, pPol, encoding the polio polymerase (19). Lane M, molecular weight markers (68 kd - bovine serum albumin; 43 kd - ovalbumin; 29 kd -carbonic anhydrase; 18 kd - beta-lactoglobulin; 14 kd -lysozyme; 6 kd - bovine trypsin inhibitor).

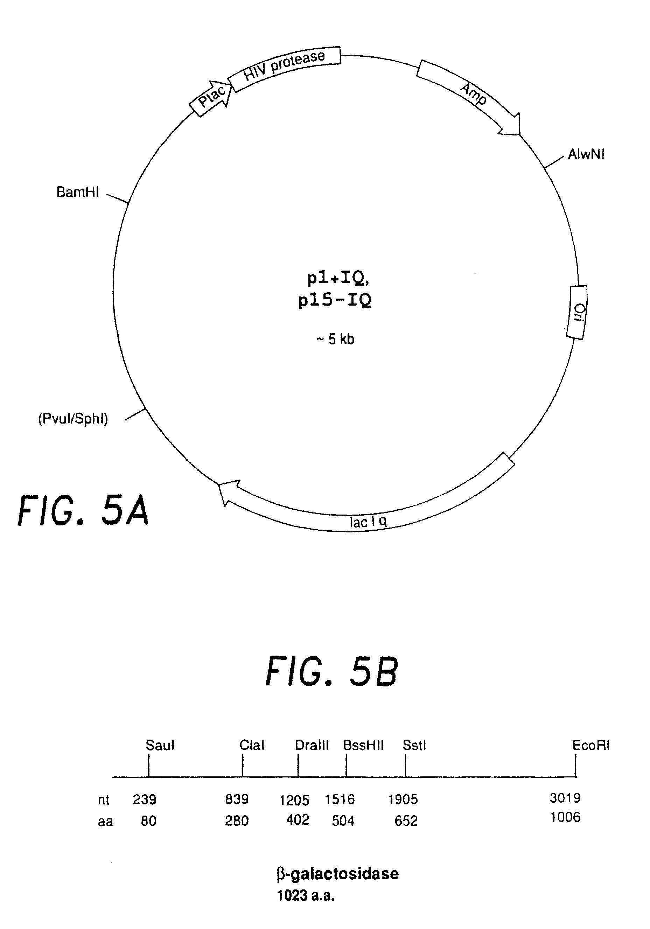

Figure 5, Panel A depicts the HIV protease plasmids p1+IQ and p15-IQ which encode wild type and mutant HIV protease, respectively, under tac promoter control. E. coli strain MC1061 harboring these plasmids is used as a source of HIV protease for in vitro cleavage of β-galactosidase. For monitoring cleavage in vivo , the β-galactosidase/cleavage cassette genes are excised from the pZM plasmid series with StuI and BamHI, and ligated into the BamHI site of p1+IQ or p15-IQ using BamHI linkers, so that HIV protease and β-galactosidase are co-expressed in the same E. coli cell. Panel B depicts the insertion of the HIV protease cleavage site into β-galactosidase. Oligonucleotides encoding the HIV protease cleavage site are inserted into the indicated restriction sites of the β-galactosidase gene. The nucleotide (nt) and amino acid (aa) positions with β-galactosidase are indicated.

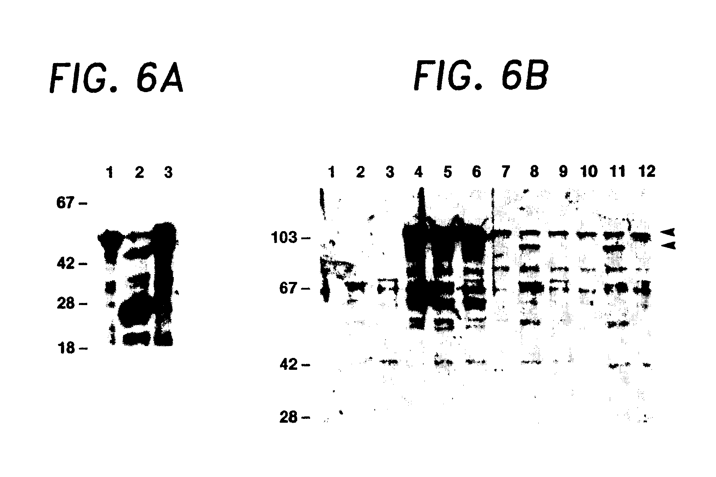

Figure 6, Panel A depicts the in vitro cleavage of gag polyprotein by HIV protease. Gag polyprotein is incubated with buffer (lane 1), or with wild type (lane 2) or mutant (lane 3) HIV protease, followed by electrophoresis and autoradiography. Molecular weight markers are shown on the left (67 kd -bovine serum albumin; 42 kd - ovalbumin; 28 kd -carbonic anhydrase; 18 kd - beta-lactoglobulin). Panel B depicts the in vitro cleavage of β-galactosidase/cleavage cassette proteins by HIV protease. Extracts of MC1061 cells harboring HIV protease plasmid are incubated with extracts harboring β-galactosidase, followed by Western blotting with anti-β-galactosidase. Lane 1, MC1061 (no plasmid). Lane 2, wild type HIV protease extract. Lane 3, mutant HIV protease extract. MC1061/pZM1 extract (lane 4) is incubated with wild type (lane 5) or mutant (lane 6) HIV protease. MC1061/pZM-Sau extract from two independent clones (lanes 7 and 10) is incubated with wild type (lanes 8 and 11) or mutant (lanes 9 and 12) HIV protease. The upper arrow on the right indicates β-galactosidase; the lower arrow on the right indicates the cleavage product from pZM-Sau. Molecular weight markers are shown on the left (103 kd - phosphorylase b; 67 kd - bovine serum albumin; 42 kd - ovalbumin; 28 kd - carbonic anhydrase).

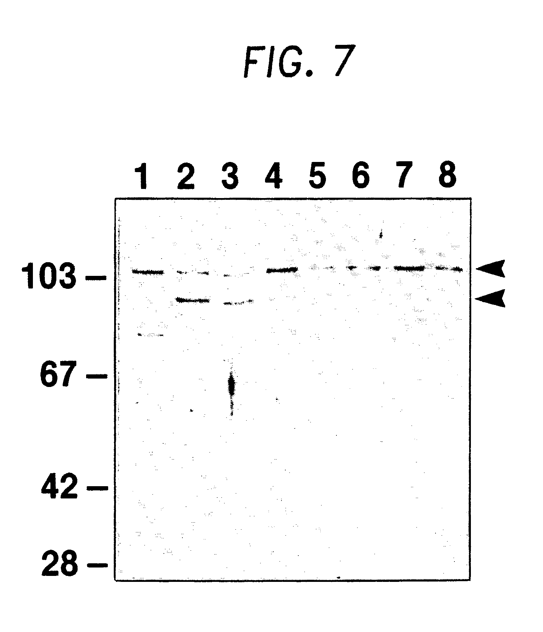

Figure 7 depicts the inhibition of cleavage of β-galactosidase by Pepstatin A. The β-galactosidase/cleavage cassette protein from MC1061 cells harboring pZM-Sau is incubated with HIV protease in the presence or absence of Pepstatin A, followed by Western blotting with anti-β-galactosidase. Lane 1, no protease. Lane 2, with protease added. Pepstatin A dissolved in dimethylsulfoxide is added to the incubation at concentrations of 0, 0.2, 1, 5, 25 and 50 μg/ml (lanes 3-8). The upper arrow on the right indicates β-galactosidase; the lower arrow on the right indicates the cleavage product from pZM-Sau. The molecular weight markers shown on the left are identical to those in Figure 6, Panel B.

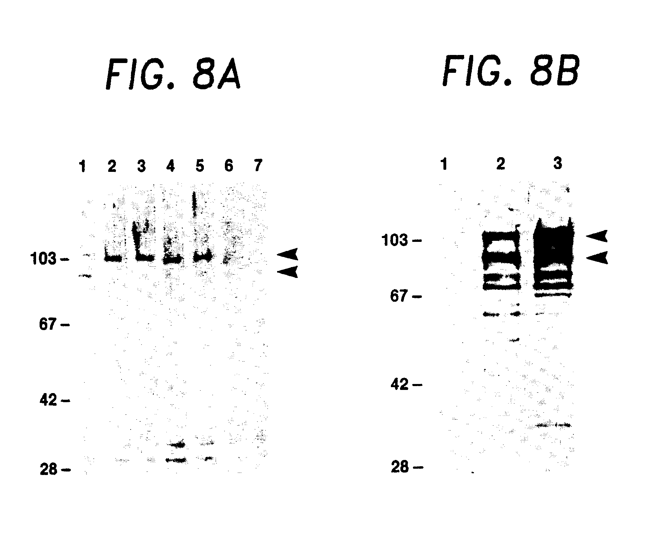

Figure 8 depicts the in vivo cleavage of β-galactosidase/cleavage cassette proteins by HIV protease. Panel A: MC1061 cells harboring p1+IQSau (lane 1), p1+IQ-ClaA (lane 2), p1+IQ-ClaB (lane 3), p1+IQ-Bss (lane 4), p1+IQ-Eco (lane 5), p1+IQ (lane 6), or without plasmid (lane 7), are grown to mid-log phase, and IPTG is added to 1 mM. After 30 minutes, cells are pelleted and resuspended in sample buffer, followed by electrophoresis and Western analysis with anti-β-galactosidase. Panel B: MC1061 cells without plasmid (lane 1) or harboring p1+IQSau (lane 2) or p15-IQSau (lane 3) are treated as in panel A. In each panel, the upper arrow on the right indicates β-galactosidase; the lower arrow on the right indicates the cleavage product from p1+IQSau. In each panel, the molecular weight markers are identical to those in Figure 6, panel B.

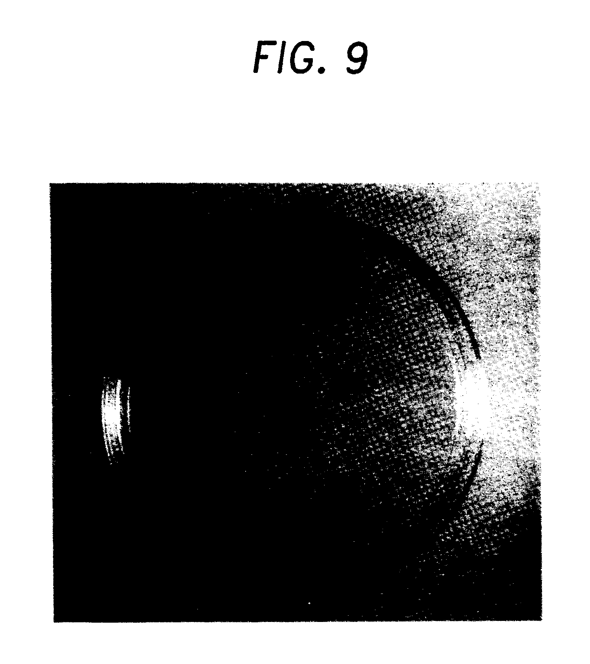

Figure 9 is a photograph which depicts the results of an in vivo cleavage assay. Samples of E. coli strain MC1061 harboring various plasmids are spotted onto plates containing the color indicator X-gal. The plasmids are pZM-1 (a positive control for β-galactosidase activity), pZM-2 (a negative control for β-galactosidase activity), pZM-Sau, p1+IQSau (labelled 1+IQB-Sau) and p15-IQSau (labelled p15-IQB-Sau). In the cleavage assay with wild-type HIV protease, constructs which cleave β-galactosidase produce a white color, while constructs which do not cleave β-galactosidase produce a blue color. Plasmid pZM-1 is constructed as described below. Plasmid pZM-2 encodes mutant, inactive β-galactosidase, and so produces white colonies. Plasmid pZM-2 is constructed by digesting plasmid pZM-1 with EcoRI and BamHI to delete the DNA sequence coding for the 18 amino acids at the carboxy-terminal end of β-galactosidase. As expected, pZM-1 produces a blue color, as do pZMSau and p15-IQSau. Plasmid pZM-2, as a negative control, produces a white color; β-galactosidase from p1+IQSau is cleaved by HIV protease and produces a white color.

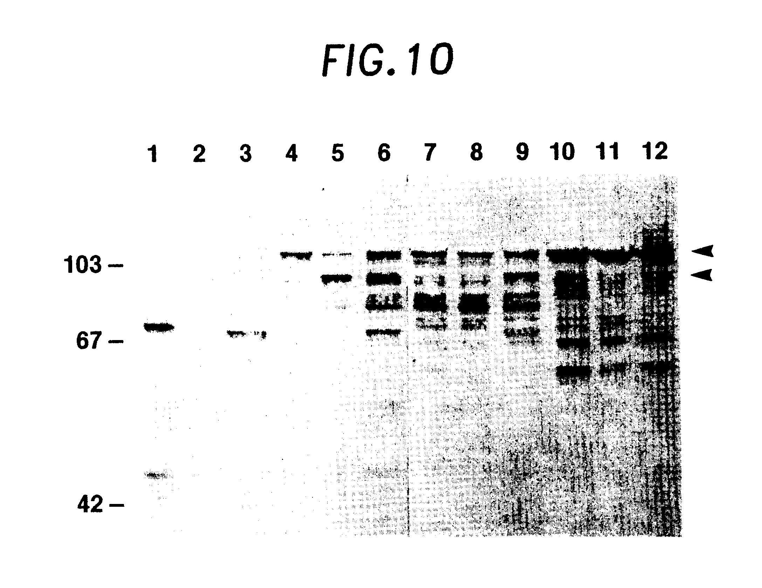

Figure 10 depicts the cleavage of polio protease cleavage cassette by polio protease in vitro . Extracts of β-galactosidase containing either the polio protease cleavage site (in MC1061/pZM3CSau) or the HIV protease cleavage site (in MC1061/pZM-Sau) are incubated with either polio or HIV protease, followed by Western analysis with anti-β-galactosidase. Lane 1, MC1061 without plasmid. Lane 2, polio 3C protease. Lane 3, HIV protease. Lane 4, MC1061/pZM3CSau unincubated. Lane 5, MC1061/pZM3CSau incubated with polio 3C protease. Lane 6, MC1061/pZM3CSau incubated with HIV protease. Lane 7, MC1061/pZM-Sau unincubated. Lane 8, MC1061/pZM-Sau incubated with polio 3C protease. Lane 9, MC1061/pZM-Sau incubated with HIV protease. Lane 10, MC1061/pZM1 unincubated. Lane 11, MC1061/pZM1 incubated with polio 3C protease. Lane 12, MC1061/pZM1 incubated with HIV protease. The upper arrow on the right indicates β-galactosidase; the lower arrow on the right indicates the cleavage product from the Sau constructs. Molecular weight markers are shown on the left (103 kd - phosphorylase b; 67 kd - bovine serum albumin; 42 kd - ovalbumin.

## DETAILED DESCRIPTION OF THE INVENTION

The present invention is directed to two screening methods for inhibitors of proteases such HIV protease, renin, trypsin, elastase and polio protease. Each screening method will first be described with respect to HIV protease and then for polio protease. The methods may also be adapted for use with other proteases in accordance with the principles of the invention.

The first screening method for inhibitors of HIV protease is directed to the construction of a plasmid which expresses HIV protease that is toxic to an E. coli strain. This toxicity, in turn, is useful to screen for agents which inhibit HIV protease.

To study the HIV protease, the BglII-DraI fragment from λBH5 (4), corresponding to nucleotides 1641-2122 of the HIV pol gene encoding the protease, is subcloned into a previously-prepared expression vector (pT7K) containing a phage T7 promoter (19), creating plasmid pPDB16 (Fig. 1). The construction of pPDB16 is carried out in E. coli strain DH5α (Bethesda Research Laboratories, Gaithersburg, Maryland), prior to transformation into the expression strain, E. coli BL21(DE3)pLysS. It is necessary to carry out this construction in a strain such as DH5α, which does not contain T7 RNA polymerase and, therefore, cannot express HIV protease, since expression of HIV protease will be shown to be toxic to the expression strain.

An E. coli strain DH5α harboring plasmid pPDB16 has been deposited with the American Type Culture Collection and has been assigned ATCC accession number 68005. Plasmid pPDB16 may be obtained through conventional techniques (20) by growing this strain in culture, breaking open cells, centrifuging and isolating the plasmid from the supernatant.

The expression strain E. coli BL21(DE3)pLysS contains a chromosomal copy of T7 RNA polymerase under the control of an inducible lacUV5 promoter, and a chloramphenicol-resistant plasmid encoding T7 lysozyme which inhibits transcription by T7 RNA polymerase in the absence of induction (21,22). Cells harboring pPDB16 are induced with the inducing agent IPTG in the presence of $^{35}$S methionine, and protein synthesis is examined by sodium dodecyl sulfate-polyacrylamide gel electrophoresis ("SDS-PAGE") using a 15 percent polyacrylamide gel (comprising 15 ml of 30% acrylamide, 1.3 ml of 2% bisacrylamide, 11.2 ml of 1M TrisCl (pH 8.7), 0.15 ml 20% SDS, 2.35 ml of $H_2O$, 25 $\mu$l N,N,N',N'-tetramethylethylenediamine and 100 $\mu$l 10% ammonium persulfate (20)), and autoradiography. The HIV DNA fragment in pPDB16 is expected to encode a primary translation product of 18 kd. Instead, as shown by SDS-PAGE, a prominent band of 10 kd is observed upon induction (Fig. 2A, lane 2). This observation is consistent with efficient autodigestion of the 18kd precursor into the mature 10 kd protease (the biologically active form) at the phenylalanine-proline bond (Fig. 1), and is in agreement with results reported previously (5,7).

The 10 kd HIV protease band detected by autoradiography is not visible by Coomassie blue staining, suggesting that only low levels of this protein are made. It has been previously determined that the absolute level of expression of a cloned gene under T7 promoter control is lower in BL21(DE3)pLysS than in the corresponding strain lacking lysozyme (BL21(DE3) (21)), due to the inactivation of T7 RNA polymerase by lysozyme (22). However, genes under T7 promoter control which are toxic to E. coli will not produce stable transformants in BL21(DE3), which lacks the lysozyme plasmid, pLysS (21).

In an attempt to increase the level of expression of the HIV protease, the use of the expression strain BL21(DE3) is investigated by applicants. The results of two transformation experiments are shown in Table I:

TABLE I

| | No. of transformants | |
|---|---|---|
| Plasmid | BL21(DE3) | BL21(DE3)pLysS |
| A. | | |
| pPDB16 | 0 | 400 |
| pPol1 | 400 | 400 |
| pPol2 | 350 | 300 |
| B. | | |
| pMae2 | 0 | 960 |
| p48Mae6 | 330 | 840 |
| pPol1 | 600 | 2400 |

Table I. Transformation of BL21(DE3) strains by HIV protease constructs. BL21(DE3) cells containing or lacking the pLysS plasmid are made competent by the standard calcium chloride procedure (20). Fifty ng of protease or control plasmid are used for each transformation. Plasmids pPDB16 and pMae2 are as previously described; plasmid pP48Mae6 encodes HIV protease with a point mutation at amino acid 29. Plasmids pPol1 and pPol2 encode the polio polymerase (19).

Whereas several plasmids as controls transform strains BL21(DE3) and BL21(DE3)pLysS equally well, plasmids pPDB16 and pMae2 encoding HIV protease do not yield transformants in BL21(DE3). These data strongly suggest that expression from pPDB16 or pMae2 is toxic to E. coli BL21(DE3). This toxicity, in turn, suggests a method of identifying agents which inhibit HIV protease.

The apparent toxicity of HIV protease could be due to its inherent enzymatic activity, or to some other effect of this protein. This question is answered by isolating mutants of the HIV protease by screening for viable transformants of BL21(DE3). To introduce mutations into the HIV protease, plasmid pPDB16 is first transformed into E. coli strain LE30 mutD (Cold Spring Harbor Laboratory, Cold Spring Harbor, New York). This strain has a $10^3$-$10^4$ higher frequency of spontaneous mutation compared to wild-type E. coli (23). Plasmid is prepared from 50,000 pooled LE30 mutD transformants, and this collective plasmid is used for the transformation of BL21(DE3). Approximately 150 colonies are obtained. These clones are examined for HIV protease production by labelling of cells with $^{35}$S methionine in the presence of IPTG, followed by SDS-PAGE using the system described previously and autoradiography. As shown in Fig. 2B, these putative mutants do not produce the 10 kd protein observed for the wild-type clone. Instead, they produce protein of either 18 kd (lanes M3, M4, M5), or protein smaller than 18 kd, but not 10 kd, (lanes M2, M6, M7). These proteins are only observed upon induction with IPTG, indicating that they are under the control of the T7 promoter. In addition, when plasmid isolated from these putative mutants is added to a DNA-dependent transcription-translation system from E. coli , protein of the same size as those seen in labelled cells is observed only in the presence of T7 RNA polymerase.

To determine if a specific genetic lesion could be correlated with the altered size of the inducible protein, the entire 500 bp HIV DNA insert of each putative mutant is sequenced. The first group of 18 BL21-(DE3) survivors is comprised of eight clones producing 18 kd protein (M3, M4, M5, M8, M9, M12, P2, and P5), seven clones producing protein smaller than 18 kd (M2, M6, M7, M10, M11, P49 and P74), and three clones which do not produce protein upon induction with IPTG (M1, P3, and P26).

The sequencing results are presented in Tables II and III. Table II presents nonsense mutants of HIV protease:

6

TABLE II

| Nonsense Mutants Of HIV Protease | |
|---|---|
| Clone | Mutation |
| P26 | CAG → UAG at nt 60 |
| M7 | CAA → UAA at nt 225 |
| P49 | CGA → UGA at nt 228 |
| M10, M11 | G insertion at nt 259, UGA at nt 293 |
| M2 | G deletion at nt 352, UAA at nt 373 |
| P74 | G insertion at nt 353, UGA at nt 383 |
| M6, P52 | CAG → UAG at nt 387 |

Table II. The entire 500 bp protease insert of pPDB16 from BL21(DE3) survivors is subjected to DNA sequence analysis, and a premature stop codon is identified. Clone P26 encodes a truncated protease of 2 kd which is not detected upon labelling of E. coli proteins, probably due to its small size. Each other clone encodes a protein smaller than 18 kd which is detected by labelling (see Fig. 2B, lanes M2, M6, M7).

Each clone which produces protein smaller than 18 kd contains a premature translation termination codon (Table II). The premature termination codons of clones M6, M7, and P49, result from a C -> U transition. For clones M2, M10, M11, and P74, the insertion or deletion of a G residue within a stretch of 5 or 6 G's results in a translational frameshift which should lead to termination as indicated (Table II). In each case, whether a transition or frameshift occurs, the size of the protease predicted from the DNA sequence is in excellent agreement with the size of the protein observed upon induction (Fig. 2B).

Clone P26, which apparently does not produce labelled protein upon induction with IPTG, is shown to contain a premature translation termination codon at nucleotide 61 (Table II). The truncated protein expected upon induction of this construct would be approximately 2 kd, and is too small to be resolved by the gel system used, which is based upon that of Plotch et al. (19). Clones M1 and P3 are also subjected to DNA sequence analysis, and are found to encode wild-type protease. Since whole plasmid is subjected to mutagenesis in strain LE30mutD, these clones may contain mutations outside of the HIV protease region that would prevent transcription or translation from genes under T7 promoter control, such as mutations in the promoter region itself or in the ribosome binding site (21).

For each clone which produces 18 kd protein, a single nucleotide mutation leading to a single amino acid substitution is detected by DNA sequencing (Table III). After these initial results with the first group of 18 clones are obtained, only those clones which produce 18 kd protein upon induction are examined in detail. The specific amino acid change determined for each mutant is summarized in Table III:

## TABLE III

## Missense Mutants Of HIV Protease

| Clone | Position | | Mutation | | |
|-------|----------|--|----------|--|--|
| | Nucleo-<br>tide | amino<br>acid | | | |
| P57 | 196 | -4 | UCC<br>Ser | ──> | UUC<br>Phe |
| P46 | 211 | 2 | CAG<br>Gln | ──> | CGG<br>Arg |
| P12, P37 | 214 | 3 | AUC<br>Ile | ──> | AAC<br>Asn |
| P32 | 250 | 15 | AUA<br>Ile | ──> | ACA<br>Thr |
| P29 | 270 | 22 | GCU<br>Ala | ──> | ACU<br>Thr |
| M12 | 285 | 27 | GGA<br>Gly | ──> | AGA<br>Arg |
| P48 | 292 | 29 | GAU | ──> | GGU |

| | | | | | |
|---|---|---|---|---|---|
| | | | Asp | | Gly |
| P15 | 297 | 31 | ACA | —> | GCA |
| | | | Thr | | Ala |
| P1, P5, P80 | 319 | 38 | UUG | —> | UCG |
| | | | Leu | | Ser |
| P66 | 339 | 45 | AAA | —> | GAA |
| | | | Lys | | Glu |
| M3, M5 | 346 | 47 | AUA | —> | ACA |
| | | | Ile | | Thr |
| P31, P44 | 355 | 50 | AUU | —> | ACU |
| | | | Ile | | Thr |
| M9 | 381 | 59 | UAU | —> | CAU |
| | | | Tyr | | His |
| P24 | 385 | 60 | GAU | —> | GUU |
| | | | Asp | | Val |
| P13, P47, P68 | 403 | 66 | AUC | —> | ACC |
| | | | Ile | | Thr |
| M4, P73 | 424 | 73 | GGU | —> | GAU |
| | | | Gly | | His |
| P45, P79 | 427 | 74 | ACA | —> | AUA |
| | | | Thr | | Ile |
| M8 | 433 | 76 | UUA | —> | UCA |
| | | | Leu | | Ser |
| P2, P8 | 436 | 77 | GUA | —> | GCA |
| | | | Val | | Ala |
| P22 | 442 | 79 | CCU | —> | CUU |
| | | | Pro | | Leu |
| P38 | 460 | 85 | AUU | —> | ACU |
| | | | Ile | | Thr |
| P9 | 463 | 86 | GGA | —> | GAA |
| | | | Gly | | Glu |
| P50 | 465 | 87 | AGA | —> | GGA |
| | | | Arg | | Gly |
| P27 | 484 | 93 | AUU | —> | ACU |
| | | | Ile | | Thr |

Table III. Missense mutants of HIV protease. The entire 500 bp protease insert of pPDB16 from BL21(DE3) survivors producing 18 kd protease precursor (see text and Fig. 2B) is subjected to DNA sequence analysis, and a single point mutation is identified in each clone. Nucleotide and amino acid numbering are described in Fig. 1.

9

In all, 24 different point mutations are identified. Six of these mutations, at amino acids Ile 3, Ile 47, Ile 50, Gly 73, Thr 74 and Val 77, are identified in two independent clones. The mutations at Leu 38 and Ile 66 are each identified in three independent clones. Because mutations are reisolated without obtaining some other known mutations (e.g., mutation at Asp 25) (4,5), it is possible that sister plasmids from the mutagenized plasmid pool are being detected.

Some of these mutations can be grouped according to the proposed function of the amino acid within HIV protease. The triplet Asp, Thr, and Gly, is conserved throughout the family of aspartyl proteases (8) and helps to locate the active site of HIV protease at residues 25, 26, and 27. Mutations at or near these residues should render the protease enzymatically inactive by disruption of the catalytic site. The three-dimensional structure of HIV protease has been recently solved (10), and it appears that applicants' mutations at Ala 22, Gly 27, Asp 29, Thr 31, Ile 85, Gly 86, and Arg 87, are within the cleft of the active site. Applicants also find mutations in amino acids which are proposed to participate in hydrogen bonding within the protease, including Ala 22, Lys 45, Ile 47, Ile 66, Gly 73, Val 77, and Ile 85. Finally, the HIV protease is probably a dimer based on X-ray crystal structure (10,24), and applicants' mutations at Gly 27, Ile 50, and Ile 93 should interfere with dimer formation.

Loeb et al. (25) performed extensive site-directed mutagenesis between amino acids 21 and 46 of the HIV protease, and then determined processing activity. Most missense mutations abolished processing within the region, which is highly conserved among retroviral proteases. These mutations include Asp 29 -> Gly, and Thr 31 -> Ala, in agreement with applicants' results. However, in contrast to the approach of Loeb et al., applicants' method of mutant isolation appears to select only for non-functional protease. Consistent with this are 31 mutations in amino acids 21-46 which Loeb et al. determined had little or no effect on processing. None of these mutations comes through applicants' screen. Applicants' results also indicate that point mutations throughout the protease, outside of the conserved regions, can destroy the activity of the enzyme (Table III).

Krausslich et al. (26) have shown that deletion of the carboxy-terminal 17 amino acids from the 18 kd HIV protease precursor prevents autodigestion which would liberate the mature 10 kd protease and furthermore, that the precursor is unable to cleave a gag substrate in trans . Similar results were obtained by Hansen et al. (27). Four of applicants' point mutations, at Ile 85, Gly 86, Arg 87, and Ile 93, are within these 17 carboxy-terminal amino acids.

Applicants' results indicate that the mutant 18kd protease precursor is incapable of autodigestion, and is apparently not toxic to BL21(DE3). Without being bound by the ensuing discussion, applicants propose that the 10 kd mature protease exerts its toxic effect by digesting an unidentified essential E. coli protein, and that the 18 kd precursor is unable to carry out this digestion. Failure to generate the mature 10 kd protease could result from mutating an amino acid required for catalytic activity, or from disruption of the scissile Phe-Pro bond (located at amino acids -1 to +1 of the mature protease), such that autodigestion of the 18 kd precursor could not occur. Applicants propose that the mutations at Ser -4, Gln 2, and Ile 3 (Table II), render the Phe-Pro bond uncleavable. Loeb et al. (25) used site-directed mutagenesis of the pol polyprotein (which encodes the protease, reverse transcriptase, and endonuclease) to introduce substitutions at the upstream Phe-Pro bond. One class of mutants is defective at autodigestion, but can still cleave at the non-mutated reverse transcriptase and endonuclease borders. This processing results either from a small amount of undetected mature protease, or from the precursor which retained activity. Another class of mutants which fails to liberate the mature protease is also inefficient at cleavage to generate the reverse transcriptase and endonuclease. It is possible that applicants' method of mutant detection, the requirement for stable transformation of BL21(DE3), selects for 18 kd precursors which are enzymatically inactive.

To directly examine the effect of mature protease on the viability of BL21(DE3), applicants construct plasmids which encode only the 10 kd protease, bypassing the need for autodigestion of precursor (Fig. 1). As shown in Table IB, the wild-type HIV 10 kd protease plasmid (pMae2) does not transform strain BL21-(DE3) successfully, but is able to transform BL21(DE3)pLysS. Plasmid p48Mae6, encoding protease with a point mutation at amino acid 29 (Table III), is able to transform both E. coli strains.

Further evidence that the HIV protease is toxic to BL21(DE3) is provided by an examination of the survival of cultures in the presence of IPTG. In this experiment, cultures of BL21(DE3)pLysS cells harboring wild-type protease (pPDB16) or mutant protease (pP48) are grown to mid-log phase, and protease synthesis is induced with IPTG. The number of viable cells is determined by plating aliquots of cultures for colony-forming units. After 30 minutes of exposure to 1.0 mM or 0.1 mM IPTG, cells harboring wild type protease experience approximately four logs of killing compared to untreated cells (Fig. 3). In contrast, cells harboring the mutant protease are unaffected by the addition of IPTG. These data indicate that HIV protease is extremely toxic to BL21(DE3). Similarly, cells harboring plasmid encoding wild-type 10 kd protease (pMae2) are significantly more sensitive to IPTG than cells harboring plasmid encoding the corresponding mutant protease (p48Mae6).

An E. coli strain BL21(DE3) harboring plasmid pP48 has been deposited with the American Type Culture Collection and has been assigned ATCC accession number 68004. This plasmid-harboring strain may be subjected to one of the following methods to delete pP48 so as to leave E. coli strain BL21(DE3) (28,29):

1) Curing with agents which preferentially inhibit plasmid replication, such as acridine orange, acriflavin, quinacrine, or ethidium bromide: For example, BL21(DE3)/pP48 is grown in liquid culture overnight. The medium is 10g peptone and 10g yeast extract per liter, adjusted to pH 7.6 with NaOH. Then, $10^4$ cells per ml are inoculated into a series of tubes containing 10-100 µg/ml (10 µg/ml increments) of acridine orange. These cultures are incubated overnight at 37° in the dark. Cells are streaked onto LB plates from the culture containing the highest concentration of acridine orange which showed growth. individual colonies are tested for kanamycin resistance. Colonies which are kanamycin sensitive should have lost pP48, so that only E. coli strain BL21(DE3) remains.

2) Spontaneous curing: For example, BL21(DE3)/pP48 is grown to stationary phase several times in LB broth lacking kanamycin. Cells are streaked onto LB plates. Individual colonies are tested for kanamycin resistance. Colonies which are kanamycin sensitive should have lost pP48, so that only E. coli strain BL21(DE3) remains.

3) Curing with agents which inhibit DNA gyrase, such as coumermycin or novobiocin: This method is identical to acridine curing, except curing agents are used at 1 to 7 micrograms per ml.

4) Curing with an agent which inhibits RNA polymerase, such as rifampicin: This method is identical to acridine curing, except rifampicin is used at 5 to 10 micrograms per ml.

The amount of protein produced upon IPTG induction by cells harboring plasmids encoding wild-type or mutant versions of the 18 kd or 10 kd protease is examined by $^{35}$S methionine labelling and immunoprecipitation, followed by gel electrophoresis and autoradiography (Fig. 4). It is estimated that mutants P48 and P48Mae6, which encode the 18kd precursor and the 10 kd protease respectively (lanes 2 and 4), produce as much as ten-fold more protease than the corresponding wild type clones PDB16 (lane 1) and Mae2 (lane 3). The decreased amount of the wild-type protease produced compared to the mutant is probably due to the killing of cells by wild-type protease upon induction with IPTG (Fig. 3). Unexpectedly, however, the mutants still produce very low levels of the protease or precursor, despite their lack of toxicity. The mutant proteins cannot be detected by Coomassie blue staining of the corresponding gels. Since different genes are expressed at different efficiencies in the T7 system (21), there may be a barrier to overproducing HIV protease, even when its toxicity is apparently alleviated.

The HIV protease has been successfully expressed in a variety of E. coli K12 strains (5,6,7, 9,27), although at least in HB101, inhibition of growth upon induction of protease synthesis has been reported (30). The HIV protease may be especially toxic to BL21(DE3), which is an E. coli B strain. The toxicity of HIV protease to E. coli BL21(DE3)21 appears to indicate that the protease is functioning properly. In contrast to the results reported in this application, Krausslich et al. (15) report expression of HIV protease in strain BL21(DE3) lacking the lysozyme plasmid. Differences between the Krausslich et al. protease construct and that of the present invention may account for the consistent observation that BL21(DE3) cannot be transformed by pPDB16. The amino terminus of applicants' 18 kd protease is codon 1 of the pol gene preceded by a single methionine residue, whereas that of Krausslich et al. is codon 5 of the pol gene preceded by 12 amino acids from phage T7 gene 10 (26). In addition, the novel protease construct of this invention is on a plasmid encoding resistance to kanamycin, not ampicillin. However, the protease is also toxic to BL21(DE3) when introduced on a vector specifying ampicillin resistance, indicating that the putative target of the HIV protease is probably not the phosphotransferase which confers kanamycin resistance.

Recent studies with peptide substrates have identified minimal sequence requirements for cleavage by the HIV protease, and as few as seven amino acids are sufficient (11,12,16). Thus, the bacterial protein which is the putative target of the protease need bear little homology to the natural gag and pol substrates. The toxicity of the HIV protease to E. coli strain BL21(DE3) provides a method to isolate mutants of this important viral enzyme, and, more importantly, the potential to screen for protease inhibitors useful for therapy of HIV infection.

Applicants have demonstrated that HIV protease plasmids pPDB16 and pMae2 are unable to successfully transform E. coli strain BL21(DE3) (Table I). As a screen, these transformations are attempted in the presence of potential HIV protease inhibitors. Successful transformations serve to identify inhibitors of the protease. In another experiment, applicants also demonstrate that phage encoding HIV protease is unable to transfect E. coli strain BL21(DE3)F+. These transfections may also be attempted in the presence of potential protease inhibitors.

Another example of the first method for screening involves the use of BL21(DE3)pLysS transformed with HIV protease plasmids pPDB16 or pMae2. The induction of protease synthesis by IPTG kills these cells, as demonstrated in Figure 4. Potential protease inhibitors might prevent the death of induced cells.

The foregoing experiments with the first HIV protease screening method may be adapted to identify inhibitors of polio protease. The starting material for this embodiment of the invention is poliovirus type 1 (Mahoney strain) which has been deposited previously with the American Type Culture Collection and has been assigned ATCC accession number ATCC VR-59. This poliovirus strain is used to generate the polio protease 3C through a series of steps described below.

In summary, poliovirus strain 1 is first used to generate the plasmid pT7pv1-5 (31). The plasmid contains the entire cDNA sequence for poliovirus strain 1 under the control of the T7 promoter described previously. Polio protease 3C is then subcloned from this plasmid.

Specifically, in a first step, cDNA is prepared from poliovirus strain 1 and is subcloned into the T7 vector to construct plasmid pT7pv1-5 by standard techniques. The polio protease gene (nucleotides 5438-5986) is excised from plasmid pT7pv1-5 as an AsuI restriction fragment (nucleotides 5435-5953). Synthetic oligonucleotides are used to recreate the upstream and downstream coding sequences, and to provide translational start and stop codons, as well as the NdeI and BamHI restriction sites previously described with regard to Figure 1. Following this procedure yields a plasmid bearing polio protease 3C under the control of the T7 promoter.

In experiments analogous to those with HIV protease, applicants' data suggest that polio protease 3C is also toxic to E. coli strain BL21(DE3). As shown in Table IV below, the plasmid bearing polio protease 3C is unable to transform BL21(DE3) successfully, but does transform BL21(DE3)pLysS. The control plasmid pPol1 (encoding polio polymerase as described in Table I) transforms both strains with approximately equal efficiency:

TABLE IV

| No. of transformants | | |
|---|---|---|
| Plasmid | BL21(DE3) | BL21(DE3)pLysS |
| pPolio Protease 3C | 0 | 150 |
| pPol1 | 300 | 500 |

These data indicate that polio protease 3C, like HIV protease, is toxic to this E. coli strain, BL21(DE3), when placed under the control of the T7 promoter. Therefore, a screening method for polio protease inhibitors can readily be devised. In such a screen, a compound to be tested is shown to be an inhibitor of polio protease by preventing killing of the bacterial strain BL21(DE3) and permitting successful transformations of that strain by a plasmid coding for the polio protease 3C.

In turn, the results with HIV protease and polio protease 3C provide the basis for a generic screen for protease inhibitors. Of particular interest are compounds which inhibit other proteases which have been implicated in human diseases, such as human renin, trypsin and elastase.

The particular target protease is placed in a plasmid under the control of the T7 promoter. The system is next shown to be toxic to E. coli strain BL21(DE3), by failing to transform that strain even as strain BL21-(DE3)pLysS is transformed. Potential protease inhibitor compounds are then screened. Compounds which have inhibitory activity will be those which block the toxicity of the protease to BL21(DE3) and thereby permit transformations of that strain.

The second screening method for inhibitors of protease enzymes is directed to the construction of fusion proteins comprising a reporter protein (meaning an easily assayed protein) into which a protease cleavage site has been introduced. The cleavage site corresponds to a portion of a natural substrate of the protease of interest.

The reporter protein chosen is capable of being assayed for a change in color, light or activity when combined with a protease enzyme. The introduction of the cleavage site to create a protease "cleavage cassette" does not destroy the activity of the reporter protein. Thus, the fusion protein can be used in an assay system to screen for inhibitors of proteases.

When the protease enzyme is added to the fusion protein containing the protease cleavage site, the protease cleaves at the protease site, thereby inactivating the reporter protein. This loss of activity is monitored by means of a color change, light change or by directly monitoring the loss of activity. A compound to be screened is included in the assay system and is identified as an inhibitor of the protease if the reporter protein is not cleaved and remains active when monitored by any of the methods described above.

Examples of reporter proteins that are utilized in constructing the fusion proteins and which are assayed by monitoring a color change with a color indicator compound include β-galactosidase, alkaline phosphatase, β-glucuronidase and acetyltransferase. This system may be adapted for proteins specifying antibiotic resistance, such as β-lactamase. In a preferred embodiment of this invention, β-galactosidase is used as the reporter protein.

The β-galactosidase enzyme encoded by the lacZ gene of E. coli is responsible for the conversion of lactose into glucose and galactose, allowing utilization of lactose as a carbon source (32). Because β-galactosidase will also catalyze the hydrolysis of several other substrates which are converted into colored compounds, β-galactosidase has proven an invaluable tool to monitor a wide range of biological processes. For example, β-galactosidase fusions have been used to study transcriptional and translational regulation (33, and references therein). Fusion of heterologous protein to β-galactosidase can facilitate the purification of that protein (34-37). In addition, interruption of the amino terminus of β-galactosidase is the basis of identifying desired clones when using the pUC plasmid series (38).

The demonstration in this invention that foreign protein sequences can be inserted into specific regions of β-galactosidase, resulting in both the retention of β-galactosidase activity and the accessibility of the foreign sequences to proteases, is novel. In the specific case of the protease cleavage cassette, the fact that β-galactosidase can now be cleaved, and inactivated, by the cognate protease provides a simple means to monitor protease activity, and to search for inhibitors of these important enzymes.

Examples of suitable color indicator compounds include X-gal and Fast Blue RR. A protein such as β-galactosidase will react with X-gal to produce a blue color; when the protein is inactivated, no color change (white) is observed. A similar reaction of the protein with Fast Blue RR produces a red color; upon inactivation, no color change (white) is observed.

Alternatively, the protein is chosen such that it catalyzes the light-emitting chemical reaction of a protein derived from a bioluminescent or chemiluminescent organism. However, when the protein is cleaved by a protease enzyme, the reaction cannot occur, such that no light is emitted. The light may be observed with the naked eye, measured spectrophotometrically or recorded on X-ray film. In a preferred embodiment, the reporter protein used is luciferase.

Additionally, the inactivation by the protease may be monitored by an in vitro system which directly measures the activity of the protein in a reaction with a substrate.

In all of the variants of the second embodiment of this invention, the fusion protein is expressed in a suitable expression system. The host is a bacterium, yeast, insect cell, mammalian cell line, or any other suitable host expression system. In a preferred embodiment, the host is a strain of E. coli .

As illustrative of the second screening method of this invention, the construction and use of an assay system for screening for inhibitors of HIV protease with β-galactosidase and a color indicator will now be described.

The initial step is to construct a plasmid containing the gene coding for β-galactosidase. Plasmid pZM1 is constructed as follows: E. coli strain MC1061 (araD139, Δ(ara,leu)7697, ΔlacX74, galU, galK, hsdR, strA is obtained from Pharmacia. Plasmid pZM1, containing the E. coli β-galactosidase gene, is derived from pCH110 (Pharmacia). Plasmid pCH110 contains an EcoRI site which is deleted by replacing the smaller PstI/BamHI fragment from pCH110 containing the β-lactamase gene with the corresponding PstI/BamHI fragment from a variant of pBR322 which lacks the EcoRI site normally present on that fragment. Next, the EcoRI/BamHI fragment containing the carboxy terminus of the β-galactosidase gene is replaced with the oligonucleotide:

5' AATTCCAGCTGAGCGCCGGTCGCTACCATTACCAGTTGGTCTGGTGTCAAAAATAATAAG 3'

3' GGTCGACTCGCGGCCAGCGATGGTAATGGTCAACCAGACCACAGTTTTTATTATTCCTAG 5'

which recreates the carboxy terminus of β-galactosidase and places two stop codons at the end of the gene. This resulting plasmid is designated pZM1.

The next step is to construct plasmids which encode wild type and mutant HIV protease, respectively, each under tac promoter control. These plasmids, designated p1+IQ and p15-IQ, are constructed as follows: The protease genes from pMae2 (wild type) or p48Mae6 (mutant), as described previously, are excised as NdeI/BamHI fragments, made blunt by Klenow enzyme, and ligated into the Sma site of pKK223-3 (Pharmacia), creating p1+ and p15-. The SphI/AlwNI fragment from p1+ and p15-, containing the HIV protease gene, is then ligated to the PvuI/AlwNI fragment containing the lacIQ gene from pGEX2T (Pharmacia), creating p1 + IQ and p15-IQ (Fig. 5A). To delete the BamHI restriction site in the polylinker of p1 + IQ and p15-IQ, these plasmids are partially digested with BamHI, made blunt with Klenow enzyme, and ligated. The resultant plasmids, designated p1 + IQB and p15-IQB, contain a unique BaMHI site directly upstream of the tac promoter.

Thereafter, an HIV protease cleavage site is inserted into β-galactosidase by inserting an oligonucleotide into the β-galactosidase gene of plasmid pZM-1. Studies from several laboratories using synthetic peptides which span the different cleavage sites of the gag and gag/pol polyproteins, have determined the minimal amino acid sequence requirements for cleavage by HIV protease (11,12,15,16). The p6/PR site is cleaved most efficiently in vitro , and as few as seven amino acids are sufficient for cleavage (15). Based on these minimal requirements, a series of oligonucleotides ("cleavage cassettes") are synthesized. These oligonucleotides correspond to the ten amino acids which span the p6/PR junction, and contain the appropriate cohesive ends for insertion, in frame, into six different locations of the β-galactosidase gene of plasmid pZM1 (Fig. 5B).

The decapeptide Val-Ser-Phe-Asn-Phe-Pro-Gln-Ile-Thr-Leu corresponds to the p6/PR cleavage site of the HIV gag/pol polyprotein. Six pairs of oligonucleotides ("cleavage cassettes") encoding this decapeptide are synthesized. The oligonucleotides have in common the DNA sequence shown below containing a HindIII restriction site (underlined), which encodes the decapeptide in each oligonucleotide pair:

**Val Ser Phe Asn Phe Pro Gln Ile Thr Leu**

**5' GTA AGC TTT AAC TTC CCT CAG ATC ACT CTG 3'**

**3' CAT TCG AAA TTG AAG GGA GTC TAG TGA GAC 5'**

Each pair contains additional nucleotides specifying different cohesive ends designed for insertion, in the proper reading frame, into the unique BssH2, ClaI, DraIII, EcoRI, SauI or SstI restriction sites of the E. coli β-galactosidase gene of pZM1 (Fig. 5B). The six different sites in β-galactosidase are selected because they are unique restriction sites within pZM1, and the cleavage cassette can be inserted by a simplified cloning procedure described below. The complete DNA sequences of the six pairs of oligonucleotides are as follows (with the HIV cleavage site set off by brackets):

ECO1/2 (xRI cohesive ends):

```
5' AA TTT [GTA AGC TTT AAC TTC CCT CAG ATC ACT CTG] C        3'
3'      A [CAT TCG AAA TTG AAG GGA GTC TAG TGA GAC] GTT AA 5'
```

SST1/2 (xSstI cohesive ends):

```
5'      G [GTA AGC TTT AAC TTC CCT CAG ATC ACT CTG] CAG CT 3'
3' TC GAC [CAT TCG AAA TTG AAG GGA GTC TAG TGA GAC] G       5'
```

CLA1/2 (xClaI cohesive ends)

```
5'      C [GTA AGC TTT AAC TTC CCT CAG ATC ACT CT ]       3'
3'        [ AT TCG AAA TTG AAG GGA GTC TAG TGA GAG] C      5'
```

DRA1/2 (xDraIII cohesive ends)

```
5'      G [GTA AGC TTT AAC TTC CCT CAG ATT ACG CT ]       3'
3' C GAC [CAT TCG AAA TTG AAG GGA GTC TAA TG      ]       5'
```

SAU1/2 (xMstIISauI cohesive ends)

```
5' T GAA [GTA AGC TTT AAC TTC CCT CAG ATT ACG CTG] AC     3'
3'      T [CAT TCG AAA TTG AAG GGA GTC TAA TGC GAC] TGA CT 5'
```

BSS1/2 (xBssH2 cohesive ends)

```
5' CG CGA [GTA AGC TTT AAC TTC CCT CAG ATT ACG CTG] A      3'
3'       T [CAT TCG AAA TTG AAG GGA GTC TAA TGC GAC] TGC GC 5'
```

To simplify the cloning procedures only the cohesive ends of each oligonucleotide pair resemble the target restriction site, so that successful ligation of the oligonucleotide into the β-galactosidase gene destroys that restriction site. Therefore, after ligation of the oligonucleotide into pZM1, the ligation mixture is digested with the target restriction enzyme to linearize any residual parent plasmid pZM1. After ethanol precipitation, the digested ligation mix is used for transformation of E. coli strain MC1061. Correct clones are identified by the presence of a new HindIII site. This method results in a high proportion of correct clones and eliminates the need for phosphatase treatment of the vector and kinasing of the oligonucleotides.

The resultant plasmids are designated pZM-Bss, pZM-Cla A, pZM-Dra, pZM-Eco, pZM-Sau, and pZM-Sst, respectively. Also constructed is plasmid pZM-Cla B, which contains the cleavage cassette in the reverse orientation encoding the unrelated decapeptide Glu-Ser-Asp-Leu-Arg-Glu-Val-Lys-Ala-Tyr, which should not be recognized by HIV protease. In each case, insertion of the cleavage cassette within the β-galactosidase gene is confirmed by DNA sequencing.

To ascertain whether the desired β-galactosidase activity is retained despite the insertion of a cleavage cassette, E. coli strain MC 1061 harboring each construct is plated onto X-gal plates. In this assay, functional β-galactosidase results in the formation of blue colonies, but lack of β-galactosidase activity produces white colonies. Using procedures described below, each construct is also tested for quantitative β-galactosidase activity and for whether it is cleaved by HIV protease in vitro and in vivo . Table V sets forth the results of this assay:

TABLE V

| Beta-Galactosidase Activity of Cleavage Cassette Constructs, and Susceptibility to Cleavage by HIV Protease | | | | |
|---|---|---|---|---|
| Plasmid | Colony color on X-gal plates | beta-galactosidase activity (units)[1] | Cleavage by HIV protease[2] | |
| | | | in vitro | in vivo |
| pZM-Bss | Blue | 5 | no | no |
| pZM-Cla A | Blue | 10 | no | no |
| pZM-Cla B | Blue | 163 | no | no |
| pZM-Dra | White | 0 | no | n.d.[3] |
| pZM-Eco | Blue | 2 | no | no |
| pZM-Sau | Blue | 63 | yes | yes |
| pZM-Sst | White | 0 | no | n.d. |
| pZM1 | Blue | 1179 | no | n.d. |
| none | White | 0 | - | - |

[1] beta-galactosidase activity is determined by hydrolysis of ONPG
[2] cleavage of beta-galactosidase by HIV protease is determined by Western analysis (see Figures 6 and 8)
[3] n.d. = not done

Insertion of the cleavage cassette at the SauI, ClaI, BssH2 or EcoRI sites results in blue colonies, indicating that the enzymatic activity of β-galactosidase is retained. insertion of the cleavage cassette in the opposite orientation in the ClaI site such that it encodes a decapeptide unrelated to the HIV protease cleavage site (Table V, pZM-Cla B) also results in the formation of blue colonies. These data indicate that the β-galactosidase protein can tolerate insertions at several locations and still retain detectable enzymatic activity. Conversely, insertion at either DraIII or SstI destroys β-galactosidase activity, since colonies harboring these constructs are white.

The intensity of blue color in strains harboring the pZM plasmids Bss, Cla A, Cla B, Eco and Sau exhibits considerable variation, and in each case is less intense than that conferred by the parent plasmid pZM1. β-galactosidase activity is quantitated by hydrolysis of O-nitrophenyl-β-D-galactoside (ONPG) (39). Plates contain 5-bromo-4-chloro-3-indolyl-β-D-galactoside (X-gal) at 40 μg/ml. The assays use a modification of those described by Miller (40). Cells are grown in Luria broth to an absorbance of 0.5 (600 nanometers). Cells (0.5 ml) are pelleted and resuspended in 2 ml Z buffer (0.1 M $Na_3PO_4$, pH 7.0, 1 mM $MgSO_4$, 0.2 mM $MnSO_4$, 0.1 M β-mercaptoethanol, 0.2 mg/ml CTAB (hexadecyltrimethylammonium bromide), 0.1 mg/ml sodium deoxycholate), and incubated for five minutes at 28° C. Next, 0.6 ml of 4 mg/ml ONPG in 0.1 M $Na_3PO_4$, pH 7.0 are added, and incubated at 28° C until the solution turns yellow (10 minutes to overnight). To stop the reaction, 1.3 ml of 1 M $Na_2CO_3$ are added. The absorbance is then read at 420 and 550 nanometers. The units of β-galactosidase are calcuated using the following equation:

$$\text{units} = \frac{(A_{420} - 1.75) \times A_{550}}{t \times v \times A_{600}}$$

where A = absorbance, t = time of reaction in minutes, and v = volume of culture used in ml.

16

The results of the quantitative assay are also shown in Table V. The blue clones exhibit significantly less $\beta$-galactosidase activity than the parent plasmid pZM1, ranging from about 10 fold less (pZM-Cla B) to about 500 fold less (pZM-Eco). Without being bound by the following, it is proposed that the reduced $\beta$-galactosidase activity observed upon insertion of the cleavage cassette may result from a change in the structure of this protein which attenuates its enzymatic function, or from $\beta$-galactosidase/cleavage cassette protein which displays wild-type enzymatic activity, but is present in reduced amounts, or from a combination of these two factors. As expected, white clones pZM-Dra and pZM-Sst do not produce detectable levels of $\beta$-galactosidase activity in this assay.

The $\beta$-galactosidase/cleavage cassette proteins are next tested for susceptibility to cleavage by HIV protease in vitro . Two samples of E. coli strain MC1061, one harboring protease plasmid p1+IQ and the other plasmid p15-IQ, are grown to 0.5 absorbance units (595 nanometers). Protease synthesis is induced by addition of IPTG to 1 mM. After one hour, cells are pelleted, resuspended in 0.66 volumes M buffer (50 mM 2-(N-morpholino)ethanesulfonic acid (MES), pH 6, 1 mM dithiothreitol, and 25 mM NaCl) containing 1 mM EDTA and 1 mg/ml lysozyme, and incubated on ice for 10 minutes. Cells are pelleted and resuspended in 0.01 volume M buffer, and lysed by three freeze/thaw cycles (freezing rapidly in dry ice/ethanol, and thawing slowly in ice water). After the final thawing, the extract is centrifuged and the clear supernatant is used as a source of protease.

The same method is used to prepare extracts containing $\beta$-galactosidase from E. coli MC1061 cells harboring the pZM plasmid series described previously, except that IPTG is omitted and 0.1 volume of M buffer is used for the freeze/thaw cycle. For cleavage reactions, 20 $\mu$l of $\beta$-galactosidase extract is typically incubated with 5 $\mu$l of protease extract at 37° for one hour, followed by electrophoresis of incubation reactions on SDS-polyacrylamide gels. $\beta$-galactosidase is visualized by Western blot analysis using house anti-$\beta$-galactosidase polyclonal serum (Sigma) and the ImmunoSelect kit (Bethesda Research Laboratories). $^{35}$S-methionine-labelled control substrate corresponding to the HIV gag polyprotein (nt 109-1417) (4) is produced from the T7 promoter in E. coli strain BL21(DE3)pLysS.

An extract of E. coli strain MC1061 containing wild type protease (from p1+IQ), but not mutant protease (from p15-IQ), is able to cleave a gag polyprotein which is a natural substrate for HIV protease (Fig. 6A). Extracts of E. coli harboring $\beta$-galactosidase/cleavage cassette constructs are incubated with either wild-type or mutant HIV protease extract, and cleavage of $\beta$-galactosidase is monitored by Western analysis using anti-$\beta$-galactosidase antibodies (Fig. 6B). Native $\beta$-galactosidase encoded by pZM1 is unaffected by these incubations (lanes 4-6). Of the six $\beta$-galactosidase/cleavage cassette proteins examined, five of these (Bss, Cla A, Dra, Eco and Sst) are likewise undigested by HIV protease (Table V), as is the Cla B clone containing a reversed decapeptide insertion used as a control.

In contrast, the $\beta$-galactosidase/cleavage cassette encoded by two independent pZM-Sau clones, is cleaved by wild type HIV protease (lanes 8 and 11), producing a cleavage product of approximately 100 kd, consistent with cleavage at the SauI insertion site located 80 amino acids from the amino terminus. The other expected cleavage product of approximately 8 kd is not detected and may be unstable. Incubation of the pZM-Sau $\beta$-galactosidase/cleavage cassette with mutant HIV protease (Fig. 6B, lanes 9 and 12) fails to produce cleavage, indicating that the cleavage event is mediated by HIV protease and not by an E. coli protease.

To further examine the basis for reduced $\beta$-galactosidase activity in the $\beta$-galactosidase/cleavage cassette clones, the amount of $\beta$-galactosidase is quantitated by Western analysis, using anti-$\beta$-galactosidase antibody. It is found that the pZM-Sau construct has significantly less $\beta$-galactosidase protein compared to the parent pZM1 (Fig. 6B, compare the intensity of the $\beta$-galactosidase band in lane 4 with lanes 7 and 10). This difference is quantitated more accurately by Western analysis of different dilutions of cells harboring pZM1 and pZM-Sau, and it is determined that the pZM-Sau clone has about 10-20 fold less $\beta$-galactosidase protein than the parent clone pZM1 (data not shown). Thus, for pZM-Sau, a reduced amount of $\beta$-galactosidase protein with approximately wild-type levels of enzymatic activity, could account for the reduction of $\beta$-galactosidase activity observed on X-gal plates and in the ONPG assay (Table V).

Western analysis on the other cleavage cassette clones demonstrates similarly reduced levels of $\beta$-galactosidase protein sufficient to explain their decrease in enzymatic activity. The non-functional $\beta$-galactosidase proteins encoded by pZM-Dra and pZM-Sst are likewise present in reduced amounts. Since the native $\beta$-galactosidase encoded by pZM1 and the altered $\beta$-galactosidase/cleavage cassette proteins are expressed from identical vectors which differ only by the insertion of the cleavage cassette in the $\beta$-galactosidase gene, it appears that the $\beta$-galactosidase/cleavage cassette proteins are less stable than the native protein.

In another experiment, pepstatin A, a known inhibitor of HIV protease (34), is tested as to whether cleavage of the $\beta$-galactosidase/cleavage cassette protein pZM-Sau incubated with HIV protease is inhibited by this compound. Pepstatin A at 1 μg/ml completely inhibits the cleavage of the altered β-galactosidase, further indicating that the cleavage is due to HIV protease (Figure 7).

The results in vitro demonstrate that with the exception of the pZM-Sau construct, β-galactosidase/cleavage cassette proteins are not cleaved by HIV protease. The cleavage cassette in the protease-resistant proteins might be buried in the interior of the proteins and so be inaccessible to the protease in an in vitro assay. For this reason, it is useful next to introduce HIV protease into the same cell as these β-galactosidase/cleavage cassette proteins, to allow for co-translational cleavage in vivo .

To test for cleavage of the β-galactosidase/cleavage cassette encoded by pZM-Sau in vivo , this altered β-galactosidase gene is subcloned into the plasmids p1 + IQ and p15-IQ, which encode wild type and mutant HIV protease, respectively. Specifically, the altered β-galactosidase gene from pZM-Sau is excised as a StuI-BamHI fragment and ligated into the BamHI site of p1 + IQ and p15-IQ, creating plasmids p1 + IQSau and p15-IQSau, respectively. Samples of an E. coli strain MC1061 harboring plasmid p1 + IQSau have been deposited with the American Type Culture Collection and have been assigned ATCC accession number 68,352.

For comparison purposes, the β-galactosidase/cleavage cassette regions of pZM-Bss, pZM-Cla A, and pZM-Eco are also subcloned into p1 + IQ. The altered β-galactosidase produced by pZM-Cla B is used as a control. Since pZM-Dra and pZM-Sst produce non-functional β-galactosidase, these will not be considered further.

Total protein from cells harboring these resultant plasmids p1 + IQSau and p15-IQSau is subjected to SDS-PAGE, and cleavage of β-galactosidase is monitored by Western analysis (Fig. 8). This β-galactosidase/cleavage cassette is cleaved in vivo by wild type HIV protease (panel A, lane 1, and panel B, lane 2), but not by mutant protease (panel B, lane 3), generating a cleavage product of the same size as that observed in vitro (Fig. 6B, lanes 8 and 11). It is estimated that approximately 50-75% of the β-galactosidase/cleavage cassette protein is cleaved. These cleavage assays are done in liquid culture. The amount of cleavage which occurs in colonies is not directly quantitated, but E. coli MC1061 harboring p1 + IQSau are white on X-gal plates, whereas p15-IQSau confers blue color. These data are consistent with cleavage of the β-galactosidase/cleavage cassette by wild type, but not by mutant, HIV protease.

HIV protease does not cleave any of these other β-galactosidase/cleavage cassette proteins in vivo that are protease-resistant in vitro (Fig. 8A, lanes 2-5, and Table V). As expected, cells harboring p1 + IQBss, p1 + IQCla A, p1 + IQCla B, or p1 + IQEco are blue on X-gal plates, consistent with lack of cleavage by HIV protease.

The results of another HIV protease in vivo cleavage assay are shown in the photograph of Figure 9. Samples of E. coli strain MC1061 harboring various plasmids are spotted onto plates containing the color indicator X-gal. The plasmids are pZM-1 (a positive control for β-galactosidase activity), pZM-2 (a negative control for β-galactosidase activity), pZM-Sau, p1 + IQSau (labelled 1 + IQB-Sau) and p15-IQSau (labelled p15-IQB-Sau). In the cleavage assay with wild-type HIV protease, constructs which cleave β-galactosidase produce a white color, while constructs which do not cleave β-galactosidase produce a blue color. Plasmid pZM-2 encodes mutant, inactive β-galactosidase, and so produces white colonies. Plasmid pZM-2 is constructed by digesting plasmid pZM-1 with EcoRI and BamHI to delete the DNA sequence coding for the 18 amino acids at the carboxy-terminal end of β-galactosidase. As expected, pZM-1 produces a blue color, as do pZMSau and p15-IQSau. Plasmid pZM-2, as a negative control, produces a white color; β-galactosidase produced by p1 + IQSau is cleaved by the protease and produces a white color.

The β-galactosidase protein which has been modified by insertion of a viral protease cleavage cassette, can be used in a screen to identify inhibitors of that protease, or of any other protease whose cleavage sequence is known and can be introduced into the SauI site of β-galactosidase.

The plasmid p1 + IQSau in an appropriate E. coli strain, such as BAS849 (41) (which has a mutation in the outer membrane, rendering it more permeable to various compounds), is plated as a lawn onto an agar plate. Alternatively, a liquid culture may be used as a nutrient medium. The plate should contain sufficient IPTG to induce HIV protease synthesis, cleaving β-galactosidase and rendering the lawn colorless when assayed with β-galactosidase color indicators, such as X-gal (40), Fast Blue RR (42) or others. Possible inhibitors of the protease are spotted onto the lawn, which is then overlayed with the indicator.

A protease inhibitor will inactivate the protease, preventing cleavage of β-galactosidase. The β-galactosidase then reacts with the indicator, causing the spot to become colored (blue in the case of X-Gal, red in the case of Fast Blue RR). Thus, protease inhibitors are identified.

A similar screening method may be used where a light-emitting reaction is monitored, rather than a color change. The cleavage cassette is inserted into a protein such as luciferase (Clontech Laboratories, Inc., Palo Alto, CA), rather than β-galactosidase. In the absence of a protease enzyme, the luciferase/cleavage cassette protein catalyzes the light-emitting chemical reaction. In the presence of luciferin and ATP, an enzyme-bound luciferyl-adenylate complex is formed, which is followed by oxidative decarboxylation. The reaction products are $CO_2$, oxyluciferin and AMP, together with the emission of light. The light emission may be observed with the naked eye, measured spectrophotometrically or recorded on X-ray film.

When a protease enzyme is present, it cleaves at the protease enzyme cleavage site, thereby blocking the reaction catalyzed by luciferase, such that no light is emitted. In a screen for protease inhibitors, a test compound is included in the assay system. If the compound prevents cleavage by the protease, the reaction proceeds and light is emitted, thereby identifying the compound as a protease inhibitor.

As described above, in both the color indicator and light-emitting reaction in vivo assays, the expression system contains genes coding for the β-galactosidase/cleavage cassette protein and HIV protease. The expression system expresses both proteins used in the assay system.

The β-galactosidase/cleavage cassette protein can also be assayed directly in a test tube. In this in vitro assay, the expression system contains only the gene coding for the β-galactosidase/cleavage cassette protein. The HIV protease is generated separately and kept apart from the fusion protein until the time of the assay. At that time, the purified β-galactosidase/cleavage cassette protein is mixed with HIV protease and a potential protease inhibitor in solution. The β-galactosidase activity is monitored by hydrolysis of ONPG, using a spectrophotometer. A protease inhibitor will increase β-galactosidase activity over that observed in a control experiment with only the fusion protein and HIV protease.

The foregoing screening methods of the second embodiment of this invention may also be used to screen for inhibitors of other proteases, such as polio 3C protease. First, a β-galactosidase/cleavage cassette protein is constructed. For example, an oligonucleotide encoding the polio 3C protease cleavage site Met-Glu-Ala-Leu-Phe-Gln-Gly-Pro-Leu-Gln-Tyr-Lys-Asp (43) is inserted, in frame, into the SauI site of β-galactosidase, creating pZM3CSau. Cells harboring this plasmid are blue, consistent with results for pZM-Sau, which contains the HIV protease cleavage cassette (Table V). These data further indicate that the SauI site is an appropriate site for insertion of heterologous protein sequences without destroying β-galactosidase activity.

As shown in Figure 10, this fusion protein is cleaved by polio 3C protease (lane 5), which is expressed in BL21(DE3)pLysS in similar fashion as Nicklin et al. (44), but not by HIV protease (lane 6), as expected. This construct may then be used to screen for inhibitors of polio 3C protease, using the methods described previously.

## Bibliography

1. T. Imai, et al., J. Biochem. , 100 , 425-432 (1986).
2. D. W. Cox and H. Levison, Am. Rev. Respir. Dis. , 137 , 371-375 (1988).
3. R. J. Albin, et al., Am. Rev. Respir. Dis. , 135 , 1281-1285 (1987)
4. L. Ratner, et al., Nature , 313 , 277-284 (1985).
5. S. F. J. Le Grice, et al., EMBO , 7 , 2547-2553 (1988).
6. N. E. Kohl, et al., Proc. Natl. Acad. Sci. U.S.A. , 85 , 4686-4690 (1988).
7. W. G. Farmerie, et al., Science , 236 , 305-308 (1987); C. Debouck, et al., Proc. Natl. Acad. Sci. U.S.A. , 84 , 8903-8906 (1987); M. C. Graves, et al., Proc. Natl. Acad. Sci. U.S.A. , 85 , 2449-2453 (1988).
8. L. H. Pearl and W. R. Taylor, Nature , 329 , 351-354 (1987).
9. S. Seelmeier, et al., Proc. Natl. Acad. Sci. U.S.A. , 85 , 6612-6616 (1988).
10. M. A. Navia, et al., Nature , 337 , 615-620 (1989).
11. S. Billich, et al., J. Biol. Chem. , 263 , 17905-17908 (1988).
12. M. L. Moore, et al., Biochem. Biophys. Res. Commun. , 159 , 420-425 (1989).
13. J. J. Blumenstein, et al., Biochem. Biophys. Res. Commun. , 163 , 980-987 (1989).
14. G. B. Dreyer, et al., Proc. Natl. Acad. Sci. U.S.A. , 86 , 9752-9756 (1989).
15. H. Krausslich, et al., Proc. Natl. Acad. Sci. U.S.A. , 86 , 807-811 (1989).
16. P. L. Darke, et al., Biochem. Biophys. Res. Commun. , 156 , 297-303 (1988).
17. E. D. Matayoshi, et al., Science , 247 , 954-958 (1990).
18. A. Billich, et al., J. Biol. Chem. , 371 , 265-272 (1990).
19. S. J. Plotch, O. Palant, Y. Gluzman, J. Virol. , 63 , 216-225 (1989).
20. T. Maniatis, et al., Molecular cloning: a laboratory manual , Cold Spring Harbor Laboratory, Cold Spring Harbor, N.Y., pages 86-94, 250-251, 348 (1982).
21. F. W. Studier and B. A. Moffatt, J. Mol. Biol. , 189 , 113-130 (1986).

22. B. A. Moffatt and F. W. Studier, Cell , 49 , 221-227 (1987).

23. R. G. Fowler, et al., Molec. gen. Genet. , 133 , 179-191 (1974).

24. T. D. Meek, et al., Proc. Natl. Acad. Sci. U.S.A. , 86 , 1841-1845 (1989).

25. D. D. Loeb, et al., J. Virol. , 63 , 111-121 (1989).

26. H. Krausslich, et al., J. Virol. , 62 , 4393-4397 (1988).

27. J. Hansen, et al., EMBO , 7 , 1785-1791 (1988).

28. R. F. Schleif and P.C. Wensink, Practical Methods in Molecular Biology , Springer-Verlag, New York, pages 19-20 (1981).

29. L. Caro, et al., Methods in Microbiology , 17 , P.M. Bennett & J. Grinsted, eds., Academic Press, New York, pages 114-122 (1984)

30. P. L. Darke, et al., J. Biol. Chem. , 264 , 2307-2312 (1989).

31. S. Van Der Werf, et al., Proc. Natl. Acad. Sci. U.S.A. , 83 , 2330-2334 (1986).

32. F. Jacob and J. Monod, J. Mol. Biol. , 3 , 318-356 (1961).

33. T. J. Silhavy, et al., "Experiments with Gene Fusions", Cold Spring Harbor, N.Y. (1984).

34. M. Koenen, et al., EMBO J. , 1, 509-512 (1982).

35. U. Ruther and B. Muller-Hill, EMBO J. , 2 , 1791-1794 (1983).

36. J. Germino and D. Bastia, Proc. Natl. Acad. Sci. U.S.A. , 81, 4692-4696 (1984).

37. S. Scholtissek and F. Grosse, Gene , 62 , 55-64 (1988).

38. C. Yanisch-Perron, et al., Gene , 33 , 103-119 (1985).

39. D. M. Rothstein, et al., Proc. Natl. Acad. Sci. U.S.A. , 77 , 7372-7376 (1980).

40. J. Miller, "Experiments in Molecular Genetics", Cold Spring Harbor, N.Y. (1972).

41. B. A. Sampson, et al., Genetics , 122 , 491-501 (1989).

42. R. K. Elespuru and M. B. Yarmolinsky, Environmental Mutagenesis , 1 , 65-78 (1979).

43. P. V. Pallai, et al., J. Biol. Chem. , 264 , 9738-9741 (1989).

44. M. J. H. Nicklin, et al., J. Virol. , 62 , 4586-4593 (1988).

## Claims

1. A method for screening for compounds which inhibit protease enzymes, which comprises the steps of:

(a) constructing a plasmid having a DNA sequence which encodes a protease under the control of a T7 promoter;

(b) selecting a bacterial expression strain which is not transformed by said plasmid because of the toxicity of the protease to the bacterial strain; and

(c) adding a compound to be screened together with the plasmid to the bacterial strain such that, if the bacterial strain is not killed and produces transformants, the compound is identified as an inhibitor of the protease.

2. The method of claim 1, wherein the bacterial strain is E. coli BL21(DE3) and the plasmid has a DNA sequence which encodes human immunodeficiency virus protease.

3. The method of claim 1, wherein the plasmid is pPolio protease 3C and the plasmid has a DNA sequence which encodes polio protease 3C.

4. A method for screening for compounds which inhibit human immunodeficiency virus protease, which comprises the steps of:

(a) constructing a plasmid having a DNA sequence which encodes human immunodeficiency virus protease under the control of a T7 promoter;

(b) selecting a bacterial strain transformed by said plasmid, which strain is killed by the induction of protease synthesis by the inducing agent isopropyl-$\beta$-D-thiogalactopyranside; and

(c) adding a compound to be screened together with isopropyl-$\beta$-D-thiogalactopyranoside to the transformed bacterial strain such that, if the induced cells are not killed, the compound is identified as an inhibitor of the human immunodeficiency virus protease.

5. The method of claim 4, wherein the bacterial strain is E. coli BL21(DE3)pLysS.

6. A method of preventing the expression of enzymatically active 10 kd human immunodeficiency virus protease, which comprises: (a) introducing a mutation into the DNA sequence which encodes the 18 kd protease precursor of the 10 kd protease, thereby preventing autodigestion of the precursor which would otherwise liberate the mature, enzymatically active 10 kd protease; or (b) introducing a mutation into the DNA sequence which encodes the 10 kd protease.

7. A method of producing non-functional 10 kd human immunodeficiency virus protease, which comprises modifying the DNA sequence which encodes the 18 kd protease precursor of the 10 kd protease, such that the scissile Phe-Pro bond located at amino acids -1 to 1 of the mature 10 kd protease is disrupted.

8. A method for screening for compounds which inhibit protease enzymes, which comprises the steps of:

(I)

(a) selecting a reporter protein which is capable of being assayed by reacting with a color indicator compound to produce a color change;

(b) inserting into the gene coding for said protein a heterologous DNA sequence coding for a peptide which includes a protease enzyme cleavage site;

(c) inserting into a suitable expression system the construct of step (b) and a gene coding for a protease enzyme which recognizes and cleaves the protease enzyme cleavage site of the peptide coded for by the heterologous DNA sequence of step (b);

(d) preparing an assay system which comprises:

(i) the expression system of step (c);

(ii) a color indicator compound; and

(iii) a nutrient medium; and

(e) adding a compound to be screened to the assay system, such that, if the compound inhibits the protease enzyme, the expression system produces an intact construct of step (b), the reporter protein retains its activity and color is maintained in the assay system, but if the compound is not an inhibitor, the protease cleaves the protease enzyme cleavage site of the peptide of step (b), the reporter protein is inactivated and no color is observed in the assay system; or

(II)

(a) selecting a reporter protein which is capable of catalyzing a light-emitting chemical reaction;

(b) inserting into the gene coding for said reporter protein a heterologous DNA sequence coding for a peptide which includes a protease enzyme cleavage site;

(c) inserting into a suitable expression system the construct of step (b) and a gene coding for a protease enzyme which recognizes and cleaves the protease enzyme cleavage site of the peptide coded for by the heterologous DNA sequence of step (b);

(d) preparing an assay system which comprises:

(i) the expression system of step (c); and

(ii) a compound which undergoes a light-emitting chemical reaction catalyzed by the reporter protein of step (a); and

(e) adding a compound to be screened to the assay system, such that, if the compound inhibits the protease enzyme, the expression system produces an intact construct of step (b), the reporter protein retains its catalytic activity and a light-emitting chemical reaction occurs, but if the compound is not an inhibitor, the protease cleaves the protease enzyme cleavage site of the peptide of step (b), the reporter protein is inactivated and the light-emitting chemical reaction does not occur.

9. A method of claim 8, wherein in (I) the reporter protein of step (a) is $\beta$-galactosidase, in (II) the reporter protein of step (a) is luciferase; and in either (I) or (II), the protease cleavage site of step (b) is the human immunodeficiency virus p6/PR gag /pol polyprotein cleavage site, the expression system of step (c) includes an E . coli host strain, and the protease enzyme of step (d)(i) is the human immunodeficiency virus protease.

10. A method of claim 8, wherein in either (I) or (II) the protease cleavage site of step (b) is a polio 3C protease cleavage site and the protease enzyme of step (d)(i) is polio 3C protease.

11. A method for screening for compounds which inhibit protease enzymes, which comprises the steps of:

(a) selecting a reporter protein whose activity is capable of being monitored by reacting with a substrate;

(b) inserting into the gene coding for said reporter protein a heterologous DNA sequence coding for a peptide which includes a protease enzyme cleavage site;

(c) inserting into a suitable expression system the construct of step (b);

(d) preparing an assay system which comprises:

(i) a protease enzyme which recognizes and cleaves the protease enzyme cleavage site of the peptide coded for by the heterologous DNA sequence of step (b);

(ii) the expression system of step (c) harboring the construct of step (b); and

(iii) a substrate which reacts with the reporter protein of step (a); and

(e) adding a compound to be screened to the assay system, such that, if the compound inhibits the protease enzyme, the expression system produces an intact construct of step (b), and the reporter protein retains its activity as monitored by reaction with the substrate, but if the compound is not an inhibitor, the protease cleaves the protease enzyme cleavage site of the peptide of step (b), and the reporter protein is inactivated.

12. A method of claim 11, wherein the protein of step (a) is $\beta$-galactosidase, the protease cleavage site of step (b) is the human immunodeficiency virus p6/PR gag /pol polyprotein cleavage site, the expression system of step (c) includes an E. coli host strain, the protease enzyme of step (d)(i) is the human immunodeficiency virus protease, and the substrate of step (d)(iii) is O-nitrophenyl-$\beta$-D-galactoside.

13. A method of claim 11, wherein the protease cleavage site of step (b) is a polio 3C protease cleavage site and the protease enzyme of step (d)(i) is polio 3C protease.

14. A plasmid selected from the group consisting of plasmids designated pPDB16, pMae2, p1+IQSau and pZM3CSau.

15. A bacterial strain harboring a plasmid selected from the group consisting of E. coli strain DH5$\alpha$ harboring plasmid pPDB16 (ATCC 68005), E. coli strain BL21(DE3) harboring plasmid pP48 (ATCC 68004) and E. coli strain MC1061 harboring plasmid p1+IQSau (ATCC 68,352).

SEQUENCE LISTING

INFORMATION FOR SEQ ID NO: 1:

SEQUENCE TYPE: Oligonucleotide

SEQUENCE LENGTH: 60 nucleotides

FRAGMENT TYPE: Carboxy terminus of β-galactosidase plus two stop codons


AAT TCC AGC TGA GCG CCG GTC GCT ACC ATT ACC AGT TGG TCT  42

GGT GTC AAA AAT AAT AAG                                   60


INFORMATION FOR SEQ ID NO: 2:

SEQUENCE TYPE: Oligonucleotide

SEQUENCE LENGTH: 30 nucleotides

FRAGMENT TYPE: Internal coding region of the HIV gag/pol polyprotein

FEATURES: Codes for a decapeptide corresponding to the p6/PR cleavage site of the HIV gag/pol polyprotein

GTA AGC TTT AAC TTC CCT CAG ATC ACT CTG                   30

Val Ser Phe Asn Phe Pro Gln Ile Thr Leu
  1               5                   10

INFORMATION FOR SEQ ID NO: 3:

SEQUENCE TYPE: Oligonucleotide

SEQUENCE LENGTH: 36 nucleotides

FRAGMENT TYPE: Modified internal coding region of the HIV gag/pol polyprotein

FEATURES: Codes for a decapeptide corresponding to the p6/PR cleavage site of the HIV gag/pol polyprotein, and contains a cohesive end for the EcoRI restriction site of the E. coli β-galactosidase gene of plasmid pZM1.

AATTT GTA AGC TTT AAC TTC CCT CAG ATC ACT CTG C             36

INFORMATION FOR SEQ ID NO: 4:

SEQUENCE TYPE: Oligonucleotide

SEQUENCE LENGTH: 36 nucleotides

FRAGMENT TYPE: Modified internal coding region of the HIV gag/pol polyprotein

FEATURES: Codes for a decapeptide corresponding to the p6/PR cleavage site of the HIV gag/pol polyprotein, and contains a cohesive end for the SstI restriction site of the E. coli β-galactosidase gene of plasmid pZM1.

INFORMATION FOR SEQ ID NO: 3:

SEQUENCE TYPE: Oligonucleotide

SEQUENCE LENGTH: 36 nucleotides

FRAGMENT TYPE: Modified internal coding region of the HIV gag/pol polyprotein

FEATURES: Codes for a decapeptide corresponding to the p6/PR cleavage site of the HIV gag/pol polyprotein, and contains a cohesive end for the EcoRI restriction site of the E. coli β-galactosidase gene of plasmid pZM1.

AATTT GTA AGC TTT AAC TTC CCT CAG ATC ACT CTG C          36

INFORMATION FOR SEQ ID NO: 4:

SEQUENCE TYPE: Oligonucleotide

SEQUENCE LENGTH: 36 nucleotides

FRAGMENT TYPE: Modified internal coding region of the HIV gag/pol polyprotein

FEATURES: Codes for a decapeptide corresponding to the p6/PR cleavage site of the HIV gag/pol polyprotein, and contains a cohesive end for the SstI restriction site of the E. coli β-galactosidase gene of plasmid pZM1.

G GTA AGC TTT AAC TTC CCT CAG ATC ACT CTG CAGCT                36

INFORMATION FOR SEQ ID NO: 5:

SEQUENCE TYPE:    Oligonucleotide

SEQUENCE LENGTH:    30 nucleotides

FRAGMENT TYPE:    Modified internal coding region of the HIV gag/pol polyprotein

FEATURES:  Codes for a decapeptide corresponding to the p6/PR cleavage site of the HIV gag/pol polyprotein, and contains a cohesive end for the ClaI restriction site of the E. coli β-galactosidase gene of plasmid pZM1.

C GTA AGC TTT AAC TTC CCT CAG ATC ACT CT                       30

INFORMATION FOR SEQ ID NO: 6:

SEQUENCE TYPE:    Oligonucleotide

SEQUENCE LENGTH:    30 nucleotides

FRAGMENT TYPE:    Modified internal coding region of the HIV gag/pol polyprotein

FEATURES:  Codes for a decapeptide corresponding to the p6/PR cleavage site of the HIV gag/pol polyprotein, and

contains a cohesive end for the DraIII restriction site of the E. coli β-galactosidase gene of plasmid pZM1.

G GTA AGC TTT AAC TTC CCT CAG ATT ACG CT                    30

INFORMATION FOR SEQ ID NO:  7:

SEQUENCE TYPE:   Oligonucleotide

SEQUENCE LENGTH:   36 nucleotides

FRAGMENT TYPE:   Modified internal coding region of the HIV gag/pol polyprotein

FEATURES:  Codes for a decapeptide corresponding to the p6/PR cleavage site of the HIV gag/pol polyprotein, and contains a cohesive end for the SauI restriction site of the E. coli β-galactosidase gene of plasmid pZM1.

TGAA GTA AGC TTT AAC TTC CCT CAG ATT ACG CTG AC              36

INFORMATION FOR SEQ ID NO: 8:

SEQUENCE TYPE:  Oligonucleotide

SEQUENCE LENGTH:  36 nucleotides

FRAGMENT TYPE:  Modified internal coding region of the HIV gag/pol polyprotein

FEATURES:  Codes for a decapeptide corresponding to the p6/PR cleavage site of the HIV gag/pol polyprotein, and contains a cohesive end for the BssH2 restriction site of the E. coli β-galactosidase gene of plasmid pZM1.

CGCGA GTA AGC TTT AAC TTC CCT CAG ATT ACG CTG A          36


INFORMATION FOR SEQ ID NO: 9:

SEQUENCE TYPE:  Peptide

SEQUENCE LENGTH:  10 amino acids

FRAGMENT TYPE:  Encoded by cleavage cassette in reverse orientation of plasmid pZM-ClaB

FEATURES:  Not recognized by HIV protease

Glu Ser Asp Leu Arg Glu Val Lys Ala Tyr
 1              5                  10

INFORMATION FOR SEQ ID NO: 10

SEQUENCE TYPE: Peptide

SEQUENCE LENGTH: 13 amino acids

FRAGMENT TYPE: Polio 3C protease cleavage site Met Glu Ala Leu Phe Gln Gly Pro Leu Gln Tyr Lys Asp
 1               5                  10

nucleotide:     1                    204                  500

amino acid:   − 69                  − 1, + 1             99

                      Met                    Phe Pro             Phe

**pPDB16**

Nde I  Bgl II             Mae III             Dra I  Bam H1

**pMae2**

Nde I  Mae III               Dra I  Bam H1

*FIG. 1*

# FIG. 2A    FIG. 2B

FIG. 3

# FIG. 4

## FIG. 5A

p1+IQ,
p15-IQ

~ 5 kb

Ptac

HIV protease

Amp

AlwNI

Ori

lac I q

BamHI

(PvuI/SphI)

## FIG. 5B

| | SauI | ClaI | DraIII | BssHII | SstI | | EcoRI |
|---|---|---|---|---|---|---|---|
| nt | 239 | 839 | 1205 | 1516 | 1905 | | 3019 |
| aa | 80 | 280 | 402 | 504 | 652 | | 1006 |

**β-galactosidase**
**1023 a.a.**

FIG. 6A

FIG. 6B

FIG. 7

FIG. 8A

FIG. 8B

# FIG. 9

EP 0 421 109 A2

# FIG. 10